# EUROPÄISCHE PATENTSCHRIFT

(11) **EP 1 962 929 B1**
(45) Veröffentlichungstag und Bekanntmachung des Hinweises auf die Patenterteilung: **07.09.2011**
(21) Anmeldenummer: 06818996.8
(22) Anmeldetag: 07.12.2006
(51) Int. Cl.: A61M 5/315, A61M 5/20

(54) **EINWEGINJEKTOR MIT DAUERGELADENEM FEDERENERGIESPEICHER**
ONE-WAY INJECTOR WITH CONTINUOUSLY CHARGED SPRING ENERGY STORE
INJECTEUR A UNE VOIE DOTE D'UN ACCUMULATEUR ELASTIQUE D'ENERGIE CHARGE EN PERMANENCE

(30) Priorität: 24.12.2005 DE 102005062206
(43) Veröffentlichungstag der Anmeldung: 03.09.2008
(73) Patentinhaber: LTS LOHMANN Therapie-Systeme AG, 56626 Andernach (DE)
(72) Erfinder: HOFFMANN, Hans-Rainer, 56566 Neuwied (DE)
(74) Vertreter: Schmidt, Werner
(86) Internationale Anmeldenummer: PCT/EP2006/011747
(87) Internationale Veröffentlichungsnummer: WO 2007/073839

(56) Entgegenhaltungen:
- EP-B1- 0 595 508
- WO-A-95/31235
- FR-A- 1 174 719
- GB-A- 624 958
- US-A- 3 797 488

## Beschreibung

Die Erfindung betrifft einen Einweginjektor mit einem Gehäuse, in dem mindestens ein mechanischer Federenergiespeicher, mindestens eine - zumindest zeitweise wirkstoffbefüllbare - Zylinderkolbeneinheit, mindestens ein Kolbenbetätigungsstempel und mindestens eine Auslöseeinheit angeordnet ist.

Aus der EP 0 595 508 B1 ist ein derartiger Injektor bekannt. Er ist so konstruiert, dass sich die einzelnen Baugruppen Federenergiespeicher, Zylinderkolbeneinheit und Auslöseeinheit nicht voneinander trennen oder handeln lassen. Auch ist die Auslöseeinheit ein kompliziertes mehrteiliges System.

Ferner ist aus der DE 695 06 521 T2 eine vergleichbare Medikamenteneinspritzvorrichtung bekannt, bei der der geladene Federenergiespeicher mittels eines Brechbolzens gesichert ist. Durch das handbetätigte Anbrechen des Brechbolzens wird die den Spritzenkolben antreibende Feder freigegeben.

Des Weiteren ist in der DE 102 40 165 A1 eine Vorrichtung zum dosierten Ausstoßen eines flüssigen Wirkstoffs beschrieben. In der Vorrichtung befindet sich der Wirkstoff in einem Spritzenzylinder. Ein Spritzenkolben wird federbelastet gegen den Wirkstoff gedrückt. Die Vorschubbewegung des Spritzenkolbens wird über ein Band getaktet gebremst. Der Bremsmechanismus entspricht dem Taktgeber einer mechanischen Uhr. Das Band ist hierbei um die Welle eines Ankerrades gewickelt. Die Ankerraddrehung wird über einen pendelnden Anker periodisch drehwinkelweise freigegeben.

Der vorliegenden Erfindung liegt daher die Problemstellung zugrunde, einen modular aufgebauten Einweginjektor zu entwickeln, der bei geringer Baugröße nur wenige Bauteile aufweist und bei einfacher Handhabung eine sichere Lagerung und Funktion gewährleistet.

Diese Problemstellung wird mit den Merkmalen des Hauptanspruches gelöst. Dazu umfasst der Federenergiespeicher ein vorgespanntes Federelement. Das Federelement wird von einem, die Feder zumindest bereichsweise umgebenden, Zugmittel in der vorgespannten Position gehalten. Die Auslöseeinheit umfasst ein Schneidwerkzeug, das zur Freigabe der Energie des Federenergiespeichers das Zugmittel an mindestens einer Stelle durchtrennt oder schwächt, wobei die Schwächung ein sofortiges Reißen des Zugmittels bewirkt.

Mit der Erfindung wird ein Einmalinjektor vorgestellt, dessen Kernstück ein Federenergiespeicher mit einem vorgespannten Druckfederelement ist, wobei die Federenergie über ein gespanntes Zugmittel bzw. ein Spannband oder ein Spannseil gespeichert wird. Die Federenergie kann nur über eine nicht reversible mechanische Zerstörung des Zugmittels freigegeben werden. Für die Zerstörung des Zugmittels wird ein einfaches Schneidwerkzeug benötigt. Zur Auslösung der Federenergie kann auf ein hochpräzises, vielteiliges mechanisches Rastsystem verzichtet werden. Durch die Zerstörung des Zugmittels ist zudem ein zweckentfremdendes Weiterverwenden z.B. als gefährliches Katapultspielzeug unmöglich.

Weitere Einzelheiten der Erfindung ergeben sich aus den Unteransprüchen und der nachfolgenden Beschreibung eines schematisch dargestellten Ausführungsbeispiels.
- Figur 1:: Einweginjektor mit geladenem Federenergiespeicher, jedoch ohne Zylinderkolbeneinheit;
- Figur 2:: Seitenansicht zu Figur 1 (Sechstafelprojektion)
- Figur 3:: Draufsicht zu Figur 1, jedoch ohne Auslöseknopf;
- Figur 4:: Draufsicht zu Figur 2;
- Figur 5:: Teilansicht von Figur 1 mit Gegenschneide;
- Figur 6:: Schnitt wie in Figur 2, jedoch mit Zylinderkolben- einheit und gedrückten Auslöseknopf;
- Figur 7:: Schnitt wie in Figur 6, jedoch mit entladenem Federenergiespeicher;
- Figur 8:: Teilansicht von Figur 6.

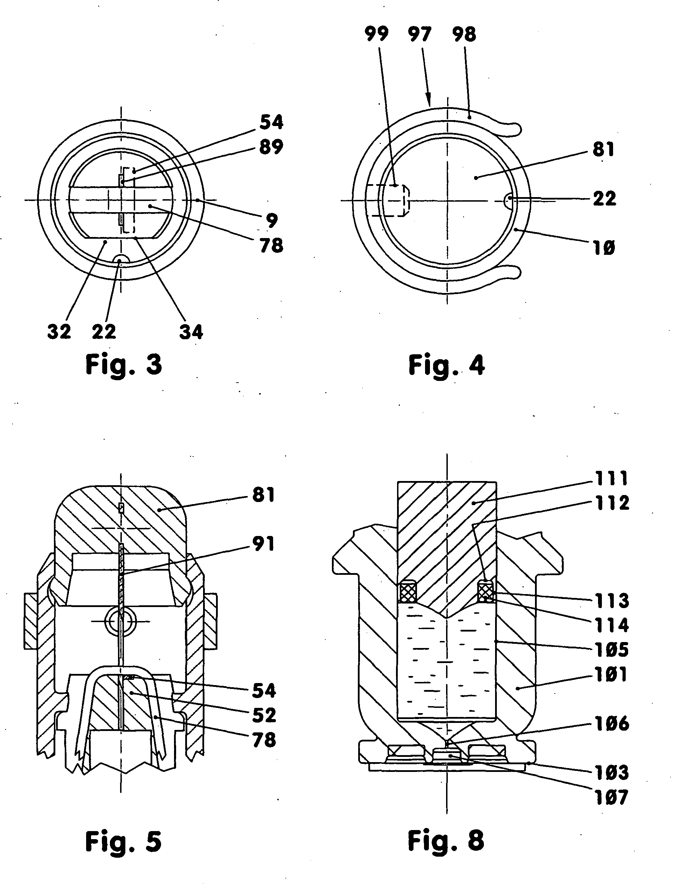

Die Darstellungen der Figuren 3, 4, 5 und 8 sind vergrößert.

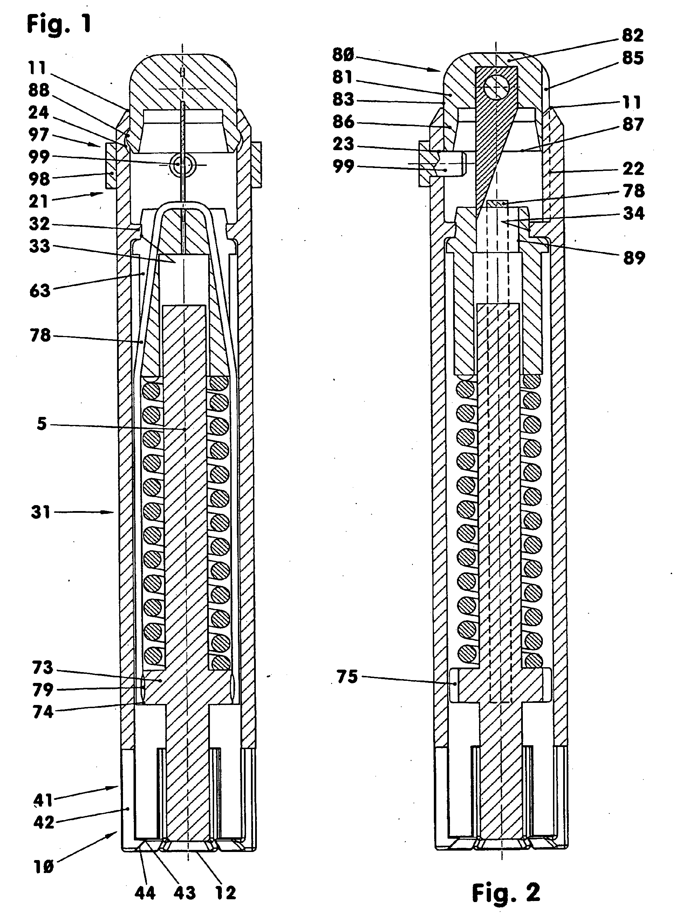

Die Figuren 1 und 2 zeigen einen Einweginjektor mit einem dauergeladenen Federenergiespeicher (50). Letzterer besteht im Wesentlichen aus einer mittels eines Spannbandes (78) vorgespannten Schraubendruckfeder (77). Der Federenergiespeicher (50) ist zusammen mit einer Auslöseeinheit (80) in einem Gehäuse (10) untergebracht. Das Gehäuse (10) lagert ggf. auch eine - ein zu verabreichendes Medikament (1) aufnehmende - Zylinderkolbeneinheit (100).

Das Gehäuse (10) besteht großteils aus einem z.B. zylindrischen Rohr, das in drei Funktionsbereiche (21, 31, 41) aufgeteilt ist. Nach den Figuren 1 und 2 ist der obere Bereich der Auslösebereich (21). An ihn schließt sich der Mantelbereich (31) an. Zwischen beiden Bereichen ist ein nach innen ragender Mittenflansch (32) angeordnet. Der Mittenflansch (32) hat eine zentrale Flanschausnehmung (33), die geometrisch durch einen Kreisabschnitt (34) von einem Kreisquerschnitt abweicht. Das Vorhandensein des Kreisabschnitts (34) dient einem weiter unten beschriebenen verdrehsicheren Zusammenbau. Die Flanschausnehmung (33) ist vom Mantelbereich (31) her zumindest bereichsweise angefast.

Im Auslösebereich (21) des Gehäuses (10) befindet sich zwischen der - nach Figur 2 - oberen Stirnfläche (11) und dem Mittenflansch (32) eine querliegende Bohrung (23). Letztere dient der Aufnahme einer Auslösesperre (97). Gegenüber der Bohrung (23) trägt die Innenwandung des Gehäuses (10) einen Verdrehsicherungssteg (22). Der Verdrehsicherungssteg (22), der in den Figuren 3 und 4 in der Draufsicht zu sehen ist, soll einen verdrehsicheren Einbau eines Auslöseknopfes (81) der Auslöseeinheit (80) ermöglichen. Die Außenkante der Stirnfläche (11) ist aus ergonomischen Gründen angefast. Oberhalb der Bohrung (23) befinden sich um jeweils 90 Winkelgrade versetzt zwei z.B. kugelkalottenförmige Vertiefungen (24), vgl. Figur 1, zur Verrastung des Auslöseknopfes (81).

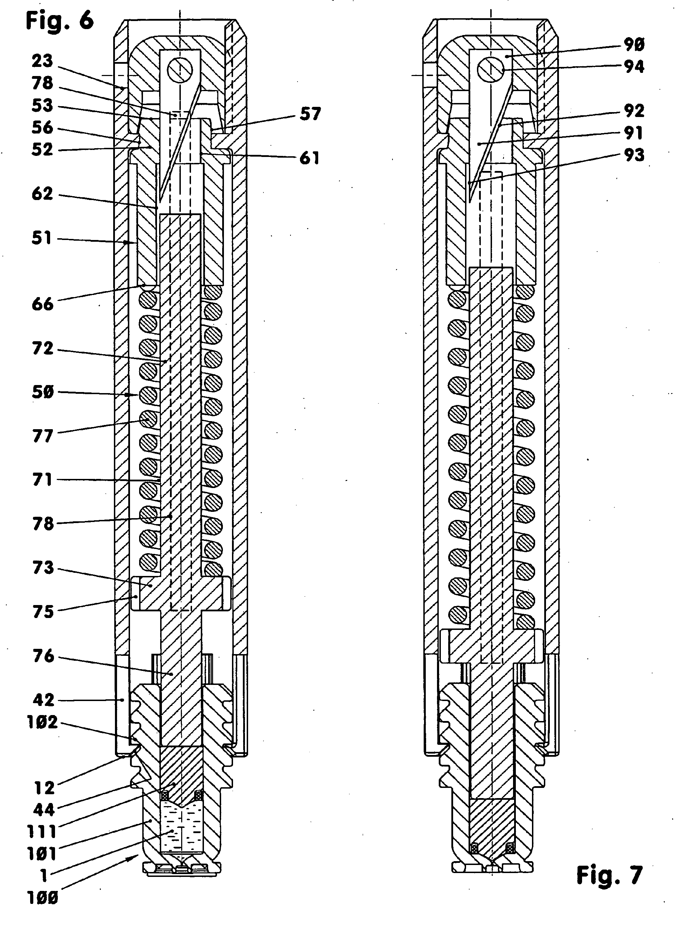

Im Gehäuse (10) befindet sich unterhalb des Mantelabschnitts (31) der Fixierbereich (41) zur Aufnahme der einbaubaren Zylinderkolbeneinheit (100), vgl. auch Figuren 6 und 7. Der Fixierbereich (41) umfasst z.B. sechs Federhaken (42), die jeweils in einer nach innen gerichteten Hakenspitze (43) enden. Die Hakenspitzen (43) haben zur unteren Gehäusestirnseite (12) hin eine sich über die gesamte Hakenstärke erstreckende Anfasung (44). Die Länge und die Federrate der Federhaken (42) ist so dimensioniert, dass die für die Funktion des Einweginjektors erforderlichen Einbauten (50, 100) ohne plastische Verformung der Federhaken (42) eingebaut werden können.

Einer dieser Einbauten ist die Zylinderkolbeneinheit (100), vgl. Figur 6. Sie besteht aus einem Zylinder (101) und einem Kolben (111). Der Zylinder (101) ist ein z.B. dickwandiger Topf, dessen ggf. zylindrische Außenwandung beispielsweise fünf umlaufende Rastrippen (102) trägt. Die Summe der Rastrippen (102) hat im Querschnitt z.B. ein Sägezahnprofil, wobei die Teilung zwischen den zahnartigen Rastrippen (102) äquidistant ist. Der maximale Durchmesser der Rastrippen (102) ist geringfügig kleiner als der Innendurchmesser des Gehäuses (10) im Fixierbereich (41). Der Durchmesser der zwischen benachbarten Rastrippen (102) liegenden Bereiche entspricht dem minimalen Durchmesser des Gehäuses (10) im Bereich der Hakenspitzen (43).

In der beispielsweise zylindrischen Bohrung (105) des Zylinders (101) sitzt der stangenlose Kolben (111), vgl. Figur 8. Der Kolben (111) hat an seiner vorderen, zumindest annähernd kegelig gestalteten Stirnfläche eine axiale Ringnut (112) zur Aufnahme eines Dichtringes (114) oder einer dauerelastischen Dichtmasse. Zwischen der äußeren Wandung der Ringnut (112) und der - im lastfreien Zustand - zylindrischen Außenwandung des Kolbens (111) liegt ein ringförmiger Steg (113), dessen Wandung z.B. nur 0,2 Millimeter beträgt. Die Steghöhe beträgt ein Vielfaches der Stegwandungsstärke.

Im Zentrum der Bohrung (105) des Zylinders (101), dessen Zylinderboden der Kontur der vorderen Kolbenstirnseite angepasst ist, befindet sich eine kurze zylindrische, düsenartige Bohrung (106). Ihr Durchmesser beträgt ca. 0,17 Millimeter. Diese Bohrung (106) ist zwei- bis dreimal so lang wie ihr Durchmesser. Sie mündet in einer zylindrischen Ausnehmung (107) der bodenseitigen, äußeren Stirnfläche (103) des Zylinders (101).

Zwischen dem Kolben (111) und dem Mittenflansch (32), vgl. Figur 6, ist der Federenergiespeicher (50) bzw. die Antriebseinheit des Einweginjektors angeordnet. Der Federenergiespeicher (50) umfasst eine Schraubendruckfeder (77), einen Kolbenbetätigungsstempel (71), einen Amboss (51) und ein Spannband (78). Letzteres hält diese Teile während der Energiespeicherphase zusammen.

Der Kolbenbetätigungsstempel (71) ist in drei - z.B. weitgehend zylindrische - Bereiche aufgeteilt. Der untere Bereich ist der Kolbenschieber (76). Sein Durchmesser ist etwas kleiner als der Innendurchmesser des Zylinders (105) der Zylinderkolbeneinheit (100). Die untere Stirnfläche des Kolbenschiebers (76) wirkt direkt auf den Kolben (111).

Der mittlere Bereich ist der Stempelteller (73). Der Stempelteller (73) ist eine flache, zumindest bereichsweise zylindrische Scheibe, deren Außendurchmesser wenige Zehntel Millimeter kleiner ist als der Innendurchmesser des Gehäuses (10) im Mantelbereich (31). Der Außendurchmesser des Stempeltellers (73) ist einige Millimeter größer als der Durchmesser derjenigen Öffnung, die die Spitzen (43) der Federhaken (42) bilden.

Nach Figur 1 hat der Stempelteller (73) zwei einander gegenüber liegende Rechtecknuten (74) zur Aufnahme eines Zugmittels (78), vgl. Figur 1. Der Stempelteller (73) hat z.B. zwei weitere - zwischen den Nuten (74) angeordnete - Luftzuführnuten (75).

Der an den Stempelteller (73) anschließende obere Bereich ist die z.B. zylindrische Federführungsstange (72). Ihr oberes Ende ragt mit Spiel in eine zentrale Stempelführungsbohrung (62) des Amboss (51) hinein.

Der Amboss (51), vgl. Figuren 1, 2 und 5 ist ein topfförmiges Bauteil, dessen Boden (52) eine z.B. rechteckige, schmale Ausnehmung als Messerführungsschlitz (61) aufweist. Die radiale Außenkontur des Bodens (52) hat eine bereichsweise umlaufende Verrastungsnut (56). Nach den Figuren 2, 3, 6 und 7 weist der Boden (52) im Bereich der Verrastungsnut (56) seitlich eine Abflachung (57) auf, die mit dem Kreisabschnitt (34) des Mittenflansches (32) korrespondiert.

Unterhalb der Verrastungsnut (56) hat der Amboss (51) einen Durchmesser, der nur wenig kleiner ist als der Innendurchmesser des Gehäuses (10) in diesem Bereich. Zwischen der unteren Ambossstirnfläche (66), an ihr stützt sich die Schraubendruckfeder (77) ab, und der oberen Stirnfläche (53) erstrecken sich zwei Bandführnuten (63). Der jeweilige Grund dieser Nuten (63) schließt mit der Mittellinie (5) einen Winkel von beispielsweise acht Winkelgraden ein. Die Tiefe der einzelnen Nut (63) vergrößert sich zur oberen Stirnfläche (53) des Ambosses (51) hin.

Für den Zusammenbau der vorkonfektionierbaren Antriebseinheit (50) wird die Schraubendruckfeder (77) auf die Federführungsstange (72) gesetzt, so dass sie auf dem Stempelteller (73) aufliegt. Auf das obere Ende des Kolbenbetätigungsstempels (71) wird der Amboss (51) aufgesteckt. Die Schraubendruckfeder (77) wird zwischen dem Stempelteller (73) und dem Amboss (51) auf die erforderliche Länge - z.B. in einer hier nicht dargestellten, speziellen Vorrichtung - zusammengedrückt. Über den Amboss (51) und die Schraubendruckfeder (77) wird ein Spannband (78) gelegt. Das in den Bandführnuten (63) eingelegte Spannband (78) endet im Bereich der Tellernuten (74) des Stempeltellers (73). Die beiden Enden des Spannbandes (78) werden in den Tellernuten (74) z.B. durch Kleben oder Verschweißen (79) unlösbar fixiert. Nach dem Entfernen der Antriebseinheit (50) aus der speziellen Spannvorrichtung ist diese (50) separat lagerbar oder wird unmittelbar danach in das Gehäuse (10) eines Einweginjektors eingesetzt.

Das Spannband (78) kann alternativ auch über eine formschlüssige Montagefuge am Stempelteller (73) befestigt werden. Es hat dann z.B. an jedem Ende eine Verbreiterung, die jeweils in einer entsprechenden Ausnehmung des Stempeltellers (73) sitzt.

Das Zugmittel (78) hat im Ausführungsbeispiel einen rechteckigen Vollquerschnitt. Es kann auch elliptisch, oval oder kreisrund sein. Als Werkstoff werden beispielsweise Kunststoffe auf PVC-Basis vorgeschlagen. Das Zugmittel (78) kann auch ein aus Garnen hergestelltes Seil bzw. eine Schnur sein.

Im Auslösebereich (21) des Gehäuses (10) sitzt längsverschiebbar der ein Schneidwerkzeug (90) tragende Auslöseknopf (81). Der Auslöseknopf (81) hat prinzipiell die Form einer Büchse, die aus einem Boden (82) und einer Schürze (86) besteht. Aus dem Boden (82) ragt nach innen das z.B. einschneidige Schneidwerkzeug (90) hervor.

Der Auslöseknopf (81) hat eine zylindrische Außenwandung (83), die nach Figur 2 eine in der Messerebene gelegene Halbrundnut (85) hat. In letztere ragt der Verdrehsicherungssteg (22) des Gehäuses (10) hinein. Zusätzlich hat die Außenwandung (83) im unteren Bereich - spiegelbildlich zur Messerebene - zwei radial vorstehende Nocken (88). Die Nocken (88) greifen bei dem montierten und gesicherten Auslöseknopf (81) in Vertiefungen (24) des Auslösebereichs (21) ein, vgl. Figur 1.

Die im Boden (82) des Auslöseknopfes (81) angeordnete Messerklinge (91) hat in der Seitenansicht, vgl. Figur 2, 6 und 7, z.B. die Gestalt eines Trapezes. Drei benachbarte Kanten des Trapezes schließen zwei rechte Winkel ein, während die lange, den Klinkenrücken darstellende Kante (93) mit der Schneidkante (92) z.B. einen 20°-Winkel einschließt. Der Klingenrücken (93) ist parallel zur Gerätemittellinie (5) orientiert. Er hat eine glatte Oberfläche. Nach Figur 1 ist die Schneidkante (92) beidseitig symmetrisch geschliffen. Im Montagezustand nach Figur 2 liegt der flache Klingenrücken (93) abstützend und gleitfähig an der entsprechenden Wandung der Messerausnehmung (89) an. Dabei ist die Breite der Ausnehmung (89) nur geringfügig breiter als die Wandstärke der beispielsweise aus Stahl hergestellten Messerklinge (91).

Das Schneidwerkzeug (90) sitzt z.B. umspritzt zentral im Auslöseknopf (81). Dazu hat es zur kraft- und formschlüssigen Befestigung im umspritzten Bereich des Bodens (82) eine z.B. bohrungsartige Ausnehmung (94).

Selbstverständlich kann das Schneidwerkzeug auch eine andere Gestalt und Schneidengeometrie haben. Beispielsweise kann die Schneidkante derart stetig gekrümmt sein, dass der Schneidwinkel mit zunehmendem Auslöseknopfhub zunimmt. Ferner ist es denkbar, das Schneidwerkzeug mit einer Doppelschneide auszustatten, wobei sich beide Schneiden gegenüberliegen. Bei diesem Werkzeug wird des Zugmittel (78) gleichzeitig von zwei Seiten her - also quer zur Ebene (9) - eingeschnitten.

Wird z.B. als Zugmittel (78) ein Seil verwendet, kann das Schneidwerkzeug auch doppelschneidig mit voneinander abgewandten Schneiden ausgeführt sein. In dem Fall trennt die zweiseitig schneidende Messerklinge das Seil mittig auf.

Die Schürze (86) hat einen unteren, z.B. ebenen Rand (87), der beim Betätigen des Auslöseknopfes (81) als Anschlag gegenüber dem Mittenflansch (32) des Gehäuses (10) dient. Im unbetätigten und gesicherten Zustand liegt der Rand (87) auf dem Sperrbolzen (99) der Auslösesperre (97) auf.

Die Auslösesperre (97) besteht aus dem Sperrbolzen (99) und einem diesen tragenden offenen, federnden Ring in Form einer Omegafeder (98), vgl. Figur 4. Am gesicherten Injektor sitzt die Omegafeder (98) auf der Außenwandung des Gehäuses (10). Sie umgibt die Außenwandung auf ca. 240 Winkelgraden. Der Sperrbolzen (99) steckt hierbei in der Bohrung (23). Er ragt wenige Millimeter in das Gehäuseinnere hinein, vgl. Figur 2. Ggf. ist mindestens eines der freien Enden der Omegafeder (98) mit einer Papier- oder Folienbanderole plombiert.

Zur Montage des Einmälinjektors wird die beispielsweise vormontierte Antriebseinheit (50) in das noch leere Gehäuse (10) eingesteckt. Die Antriebseinheit (50) verrastet über die Flanschausnehmung (33) verdrehsicher unlösbar im Mittenflansch (32). In einem zweiten Schritt wird die Zylinderkolbeneinheit (100) im Fixierbereich (41) eingesteckt. Unabhängig hiervon wird die Auslösesperre (97) angebracht und ggf. plombiert. Abschließend wird der mit dem Schneidwerkzeug (90) ausgestattete Auslöseknopf (81) verdrehsicher in das Gehäuse (10) eingesteckt. Der eingesteckte Auslöseknopf (81) liegt mit seinem unteren Rand auf dem Sperrbolzen (99) auf. Zusätzlich ist er über die Nocken (88) in den Vertiefungen (24) des Auslösebereichs (21) fixiert.

Der Abstand zwischen dem Auslöseknopf (81) und dem Amboss (51) ist so gewählt, dass die Spitze der Messerklinge (91) sicher in den Messerschlitz (89) hineinragt, jedoch ohne das Zugmittel (78) zu berühren, vgl. Figur 2.

Das Einstecken der Zylinderkolbeneinheit kann ggf. auch vom Nutzer durchgeführt werden.

Um das in der Zylinderkolbeneinheit aufgenommene Medikament zu applizieren, wird zunächst die Auslösesperre (97) - nach dem Zerstören der ggf. vorhandenen Plombe - durch seitliches Abziehen entfernt. Der entsicherte Einmalinjektor wird auf der Applikationsstelle normal aufgesetzt und der Auslöseknopf (81) mit Daumenkraft niedergedrückt. Durch das Niederdrücken des Auslöseknopfes (81) wird das Spannband (78) durchtrennt, vgl. Figur 6. Die abwärts bewegte Messerklinge (91) taucht hierbei das Spannband (78) zerschneidend in den Messerschlitz (89) ein. Das auf dem Amboss (51) aufliegende Spannband (78) kann der Schneidbewegung nicht ausweichen, da es durch die Wandung der Bandführnuten (63) seitenabgestützt geführt ist.

In Figur 5 ist die alternative Kombination aus Messerklinge (91) und Amboss (51) dargestellt. Die Messerklinge (91) hat eine asymmetrische Schneide, d.h. die Klinge ist nur einseitig geschliffen. Zusätzlich ist im Amboss (51) eine z.B. metallische oder keramische Gegenschneide (54), vgl. auch Figur 3, fest eingelassen. Die einander zugewandten Schneidkanten der Messerklinge (91) und der Gegenschneide (54) bilden eine Schere nach dem Prinzip des Offenschneidens. Bei dieser Lösung wird das Spannband (78) unter einem geringeren Kraftaufwand zerschnitten.

Je nach Werkstoffwahl für das Spannband (78) ist es nicht erforderlich, das Spannband (78) über seinen gesamten Querschnitt zu zerschneiden. Ggf. reicht nur ein Ritzen des Spannbandes (78), um dessen sofortiges Reißen herbeizuführen. In diesem Fall kann der erforderliche Hub des Auslöseknopfes verkürzt werden.

Unmittelbar nach dem Durchtrennen des Spannbandes (78) schiebt das Federelement (77) den Kolben (111) über den vorwärtsbewegten Kolbenbetätigungsstempel (71) in den Zylinder (101) zum Ausstoßen des Präparats (1) hinein, vgl. Figur 7. Der Ausstoßvorgang ist beendet, wenn der Kolben (111) den Boden des Zylinders (101) erreicht hat.

Ggf. ist in der Auslöseeinheit (80) zwischen dem handbetätigten Auslöseknopf (81) und dem Scheidwerkzeug (90) ein mechanisches, kraftverstärkendes Getriebe angeordnet.

### Bezugszeichenliste:

- 1: Wasser, destilliert; Medikament
- 5: Mittellinie zu Gerät und (Schraubendruckfeder)
- 9: Ebene, in der die Mittellinie des Spannbandes liegt.

- 10: Gehäuse, einteilig
- 11: Stirnfläche, oben
- 12: Stirnfläche, unten

- 21: Auslösebereich
- 22: Verdrehsicherungssteg, Stollen
- 23: Bohrung, querliegend
- 24: Vertiefungen

- 31: Mantelbereich
- 32: Mittenflansch
- 33: Flanschausnehmung
- 34: Kreisabschnitt

- 41: Fixierbereich für die Zylinderkolbeneinheit
- 42: Federhaken
- 43: Hakenspitze
- 44: Anfasung

- 50: Antriebseinheit, Federenergiespeicher
- 51: Amboss
- 52: Boden
- 53: Stirnfläche, oben
- 54: Gegenschneide, Schermesser

- 56: Verrastungsnut
- 57: Abflachung

- 61: Messerführungsschlitz, Ausnehmung
- 62: Stempelführungsbohrung
- 63: Bandführnuten

- 66: Ambossstirnfläche, unten

- 71: Kolbenbetätigungsstempel
- 72: Federführungsstange
- 73: Stempelteller
- 74: Rechtecknuten, Tellernuten
- 75: Luftzuführnuten
- 76: Kolbenschieber

- 77: Federelement, Schraubendruckfeder
- 78: Zugmittel, Spannband
- 79: Schweißzonen

- 80: Auslöseeinheit
- 81: Auslöseknopf, Druckknopf
- 82: Boden
- 83: Außenwandung, zylindrisch
- 85: Nut, Halbrundnut (Verdrehsicherung)
- 86: Schürze
- 87: Rand, unten
- 88: Nocken
- 89: Messerausnehmung, Schlitz

- 90: Schneidwerkzeug, Schneidmesser,
- 91: Messerklinge, einschneidig
- 92: Schneidkante
- 93: Klingenrücken, Kante
- 94: Ausnehmung

- 97: Auslösesperre
- 98: Omegafeder
- 99: Sperrbolzen

- 100: Zylinderkolbeneinheit
- 101: Zylinder
- 102: Rastrippen, außen
- 103: Stirnfläche
- 105: Bohrung
- 106: Bohrung, Düse
- 107: Ausnehmung in (Stirnfläche)

- 111: Kolben
- 112: Ringnut
- 113: Steg
- 114: Kolbendichtung, Dichtung

## Patentansprüche

1. Einweginjektor mit einem Gehäuse (10), in dem mindestens ein mechanischer Federenergiespeicher (50), mindestens eine - zumindest zeitweise wirkstoffbefüllbare - Zylinderkolbeneinheit (100), mindestens ein Kolbenbetätigungsstempel (71) und mindestens eine Auslöseeinheit (80) angeordnet ist,
**dadurch gekennzeichnet,**
- **dass** der Federenergiespeicher (50) ein vorgespanntes Federelement (77) umfasst,
- **dass** das Federelement (77) von einem dieses (77) zumindest bereichsweise umgebenden Zugmittel (78) in der vorgespannten Position gehalten wird,
- **dass** die Auslöseeinheit (80) ein Schneidwerkzeug (90) umfasst, das zur Freigabe der Energie des Federenergiespeichers (50) das Zugmittel (78) an mindestens einer Stelle durchtrennt oder schwächt, wobei die Schwächung ein sofortiges Reißen des Zugmittels (78) bewirkt.

2. Einweginjektor gemäß Anspruch 1, **dadurch gekennzeichnet, dass** das Gehäuse (10) einteilig ist.

3. Einweginjektor gemäß Anspruch 1, **dadurch gekennzeichnet, dass** das das Federelement (77) ganz oder bereichsweise umgebende Zugmittel (78) ein Spannband ist, dessen meisten Querschnittsmitten in einer Ebene (9) liegen, in der auch die Mittellinie (5) der Schraubendruckfeder (77) liegt.

4. Einweginjektor gemäß Anspruch 1, **dadurch gekennzeichnet, dass** das Schneidwerkzeug (90) handbetätigt ist.

5. Einweginjektor gemäß Anspruch 1, **dadurch gekennzeichnet, dass** das Schneidwerkzeug (90) ein einschneidiges Messer ist.

6. Einweginjektor gemäß Anspruch 1, **dadurch gekennzeichnet, dass** die zu durchtrennende Stelle des Zugmittels (78) auf einem Amboss (51) aufliegt, der einen Schlitz (61) zur Führung des Schneidwerkzeugs (90) umfasst.

7. Einweginjektor gemäß Anspruch 6, **dadurch gekennzeichnet, dass** eine Kante des Ambosses (51) als Gegenschneide (54) dient.

8. Einweginjektor gemäß der Ansprüche 1 und 6, **dadurch gekennzeichnet, dass** das Zugmittel (78), der Amboss (51), das Federelement (77) und ein das Federelement (77) führender Kolbenbetätigungsstempel (71) einen vormontierbaren Energiespeicher bilden.

9. Einweginjektor gemäß Anspruch 1, **dadurch gekennzeichnet, dass** die Zylinderkolbeneinheit (100) mindestens eine Komponente (1) des zu verabreichenden Präparats lagert.

10. Einweginjektor gemäß Anspruch 9, **dadurch gekennzeichnet, dass** die Komponente (1) destilliertes Wasser ist.

## Claims

1. Disposable injector with a housing (10) that contains at least one mechanical spring energy reservoir (50), at least one cylinder/piston unit (100) that can be filled at least temporarily with active substance, at least one piston-actuating rod (71) and at least one trigger unit (80), **characterized in that**
- the spring energy reservoir (50) comprises a pretensioned spring element (77),
- the spring element (77) is held in the pretensioned position by a pulling means (78) that surrounds the spring element (77) in at least some areas,
- the trigger unit (80) comprises a cutting tool (90) which, in order to release the energy of the spring energy reservoir (50), severs the pulling means (78) at at least one location, or weakens it, the weakening causing an immediate tearing of the pulling means (78).

2. Disposable injector according to Claim 1, **characterized in that** the housing (10) is in one piece.

3. Disposable injector according to Claim 1, **characterized in that** the pulling means (78), surrounding the spring element (77) completely or in some areas, is a tensioning band, and most of the cross-sectional centres of the latter lie in a plane (9) in which the centre line (5) of the helical compression spring (77) also lies.

4. Disposable injector according to Claim 1, **characterized in that** the cutting tool (90) is operated by hand.

5. Disposable injector according to Claim 1, **characterized in that** the cutting tool (90) is a knife with a single cutting edge.

6. Disposable injector according to Claim 1, **characterized in that** the location of the pulling means (78) that is to be severed bears on an anvil (51) that comprises a slit (61) for guiding the cutting tool (90).

7. Disposable injector according to Claim 6, **characterized in that** one edge of the anvil (51) serves as an opposite cutting edge (54).

8. Disposable injector according to Claims 1 and 6, **characterized in that** the pulling means (78), the anvil (51), the spring element (77) and a piston-actuating rod (71) guiding the spring element (77) form an energy reservoir that can be preassembled.

9. Disposable injector according to Claim 1, **characterized in that** the cylinder/piston unit (100) accommodates at least one component (1) of the product that is to be administered.

10. Disposable injector according to Claim 9, **characterized in that** the component (1) is distilled water.

## Revendications

1. Injecteur à une voie comprenant un boîtier (10), dans lequel sont disposés au moins un accumulateur d'énergie à ressort mécanique (50), au moins une unité cylindre-piston (100) pouvant être remplie de substance active au moins temporairement, au moins un poinçon d'actionnement de piston (71) et au moins une unité de déclenchement (80), **caractérisé en ce que**
- l'accumulateur d'énergie à ressort (50) comprend un élément à ressort précontraint (77),
- l'élément à ressort (77) est maintenu dans la position précontrainte par un moyen de traction (78) l'entourant (77) au moins en partie,
- l'unité de déclenchement (80) comprend un outil de coupe (90), qui, pour libérer l'énergie de l'accumulateur d'énergie à ressort (50), coupe ou affaiblit le moyen de traction (78) en au moins un endroit, l'affaiblissement provoquant une déchirure immédiate du moyen de traction (78).

2. Injecteur à une voie selon la revendication 1, **caractérisé en ce que** le boîtier (10) est réalisé d'une seule pièce.

3. Injecteur à une voie selon la revendication 1, **caractérisé en ce que** le moyen de traction (78) entourant complètement ou en partie l'élément à ressort (77) est une bande de serrage dont la plupart des centres en section transversale se situent dans un plan (9) dans lequel se trouve également l'axe médian (5) du ressort de compression à boudin (77).

4. Injecteur à une voie selon la revendication 1, **caractérisé en ce que** l'outil de coupe (90) est commandé manuellement.

5. Injecteur à une voie selon la revendication 1, **caractérisé en ce que** l'outil de coupe (90) est un couteau à un seul tranchant.

6. Injecteur à une voie selon la revendication 1, **caractérisé en ce que** l'endroit à couper du moyen de traction (78) se situe sur une enclume (51) qui comprend une fente (61) pour guider l'outil de coupe (90).

7. Injecteur à une voie selon la revendication 6, **caractérisé en ce qu'**une arête de l'enclume (51) sert de tranchant conjugué (54).

8. Injecteur à une voie selon les revendications 1 et 6, **caractérisé en ce que** le moyen de traction (78), l'enclume (51), l'élément à ressort (77) et un poinçon d'actionnement du piston (71) guidant l'élément à ressort (77) forment un accumulateur d'énergie prémontable.

9. Injecteur à une voie selon la revendication 1, **caractérisé en ce que** l'unité cylindre-piston (100) contient au moins un composant (1) de la préparation à administrer.

10. Injecteur à une voie selon la revendication 9, **caractérisé en ce que** le composant (1) est de l'eau distillée.
